# EUROPEAN PATENT APPLICATION

(11) **EP 1 913 909 A1**
(43) Date of publication of application: **23.04.2008**
(21) Application number: 06766727.9
(22) Date of filing: 14.06.2006
(51) Int. Cl.: A61F 7/00, A61F 7/08, A45D 44/22

(54) **HUMIDIFYING PAD**

(30) Priority: 08.08.2005 JP 2005230104
(71) Applicant: Uni-Charm Corporation, Ehime 799-0111 (JP)
(72) Inventor: MIYAZAWA, Kiyoshi, Kagawa 769-1602 (JP); TAKEUCHI, Naohito, Kagawa 769-1602 (JP); TERAOKA, Hiromi, Kagawa 769-1602 (JP); HANAJIRI, Takeshi, Kagawa 769-1602 (JP)
(74) Representative: Eke, Philippa Dianne
(86) International application number: PCT/JP2006/311959
(87) International publication number: WO 2007/017988

(57) **Abstract**

A moisturizing pad has a heat generating element sandwiched between first and second surface sheets. The heat generating element contains metal powders, salt, water and water retaining materials adapted to generate oxidation heat and thereby to convert the water to moisture vapor as the element is supplied with oxygen through an air-permeable first surface sheet. The salt and water are provided in a form of saline solution having a salt concentration of 18 - 22 % by weight and the heat generating element is impregnated with such a saline solution in a range of 17.5 to 26 % by weight of the element.

## Description

### TECHNICAL FIELD

The present invention relates to a moisturizing pad adapted to supply a user's face or a remaining skin with moisture vapor at an appropriate temperature.

### RELATED ART

There are known moisturizing pads adapted to be held in contact with a user's skin inclusive of a user's face so that the user's skin may be supplied with moisture vapor at an appropriate temperature. For example, Japanese Patent No. 3049707 (Reference 1) discloses a moisture vapor generating device for a pad of this type. The moisture generating device comprises a heat generating element containing metal powder, salt and water and a moisture-permeable sheet wrapping this heat generating element. The moisture generating device is capable of generating 0.01 mg/cm²·min or more of moisture vapor and a temperature of the moisture vapor is controlled at 50°C or lower. Japanese Unexamined Patent Application Publication No. 2004-358110 (Reference 2) discloses a deformable pad of this type which is particularly suitable for putting the pad on a user's face.

### DISCLOSURE OF THE INVENTION

### PROBLEMS TO BE SOLVED BY THE INVENTION

For a moisturizing pad to be put on a user's face or a remaining skin, there are important factors in addition to the amount as well as the temperature of moisture vapor generated from the pad. Specifically, importance should be put on a time taken until the pad is heated up to a desired temperature after the pad has been unwrapped (a rising rate of temperature) and a time for which the desired temperature is maintained (a duration of the temperature). However, neither means to accelerate the rate of rising temperature nor means to prolong the duration of the desired temperature can be found in both Reference 1 and Reference 2.

In view of the problems as have been described above, it is an object of the present invention to provide a moisturizing pad improved so that the rising rate of temperature can be accelerated and the duration of temperature can be prolonged.

### MEASURE TO SOLVE THE PROBLEMS

According to the present invention, there is provided an improvement of a pad comprising a first surface sheet to be put in contact with a user's skin, a second surface sheet lying on a side opposite to the first surface sheet and a heat generating element sandwiched between the first and second surface sheets and containing therein at least metal powders, salt, water and water retaining materials wherein, of the first and second surface sheet, at least the first surface sheet is air-permeable and the pad is wrapped with an oxygen-impermeable sheet so that the water may be vaporized under an oxidation heat generated in said heat generating element to moisturize the user's skin as the pad is unwrapped whereupon the heat generating element is supplied with oxygen through the first surface sheet.

The present invention is characterized in that salt and the water are provided in a form of saline solution with which the heat generating element is impregnated at a ratio in a range of 17.5 to 26 % by weight with respect to the heat generating element and the saline solution has a saline concentration in a range of 18 to 22 % by weight.

According to one preferred embodiment of the invention, 4 to 7 g of the moisture vapor is generated from the pad until 20 minutes elapse after the pad has been unwrapped in a room at a temperature of 20±1°C.

According to another preferred embodiment of the invention, a temperature of the pad on the side of the first surface sheet rises to 40°C within 3 minutes after the pad has been unwrapped in a room at a temperature of 20±1°C.

### EFFECT OF THE INVENTION

In the moisturizing pad according to the present invention, the heat generating element contains saline solution having a saline concentration in a range of 18 to 22 % by weight and thereby it is ensured that the temperature on the side of the first surface sheet rapidly rises to 40°C and thereafter a temperature of 40°C or higher is maintained for a long period.

With the pad implemented so that 4 to 7 g of moisture vapor can be generated within 20 minutes after the pad has been unwrapped, if the pad is put on the user's face, it is possible to supply vapor sufficient to moisturize the user's face.

The pad implemented so that a surface temperature rises to 40°C within 3 minutes after the pad has been unwrapped is useful to warm or to moisturize the user's face.

### BRIEF DESCRIPTION OF THE DRAWINGS

[FIG. 1] Fig. 1 is a plan view showing a moisturizing pad.
[FIG. 2] Fig. 2 is a sectional view taken along the line II-II in Fig. 1.
[FIG. 3] Fig. 3 is an exploded view showing a part of Fig. 2 in an enlarged scale.
[FIG. 4] Fig. 4 is a graphical diagram showing the results of measurement conducted on EXAMPLES 1, 2 and 3.
[FIG. 5] Fig. 5 is a graphical diagram showing the result of measurements conducted on EXAMPLES 4 and 5.
[FIG. 6] Fig. 6 is a graphical diagram showing the result of measurements conducted on CONTROLS.

### IDENTIFICATION OF REFERENCE NUMERALS USED IN THE DRAWINGS

- 1: moisturizing pad
- 5: user's face
- 21: first surface sheet
- 22: second surface sheet
- 26: heat generating element (moisturizing element)

### DESCRIPTION OF THE BEST MODE FOR WORKING OF THE INVENTION

Details of a moisturizing pad according to the present invention will be more fully understood from the description given hereunder with reference to the accompanying drawings.

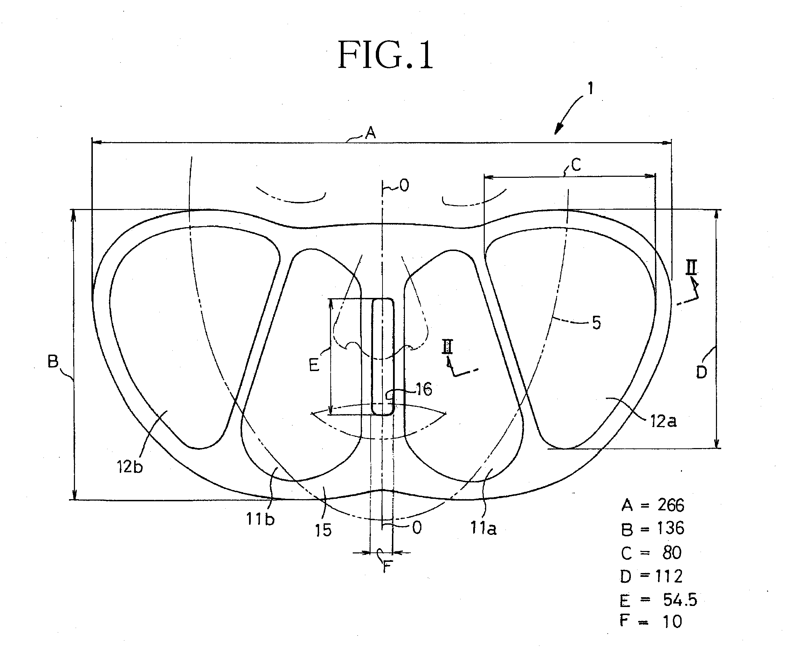

Fig. 1 is a plan view showing a face moisturizing pad as an embodiment of the invention, in which the pad 1 has been unwrapped (wrap is not shown) and developed in longitudinal and transverse directions. In Fig. 1, the user's face 5 on which the pad 1 is to be put is indicated by imaginary lines. The pad 1 has a transverse dimension of about 266 mm, a longitudinal dimension of about 136 mm and a center line O-O to be coincided with a center line of the user's face. The pad is shaped symmetrically about the center line O-O. The pad 1 has a pair of inner moisturizing sections 11a, 11b opposed to each other about the center line O-O and outside these inner moisturizing sections 11a, 11b, respectively, the pad 1 has a pair of outer moisturizing sections 12a, 12b. These moisturizing sections 11a, 11b 12a, 12b are respectively partitioned by non-moisturizing section 15 one from another and the non-moisturizing section 15 is formed on the center line O-O with a rectangular ventilator 16.

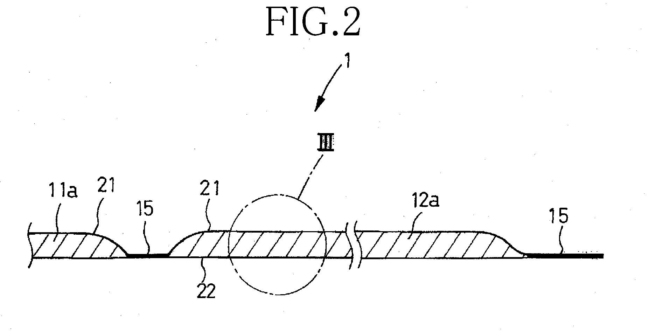

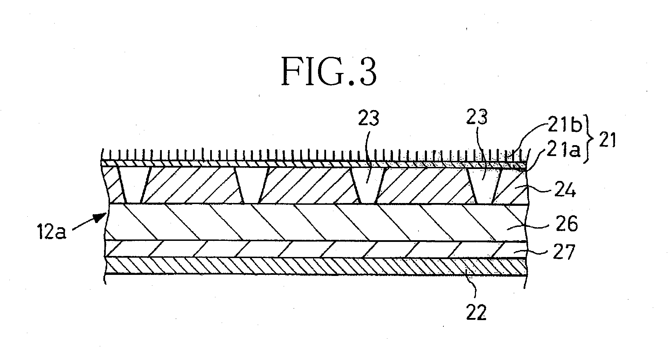

Fig. 2 is a sectional view taken along the line II-II in Fig. 1 and Fig. 3 is a view showing a part of Fig. 2 encircled by an imaginary line III in an enlarged scale. Referring to Fig. 2, the right inner moisturizing section 11a and the right outer moisturizing section 12a are substantially the same in their thicknesses as well as in their constructions and respective surfaces of these sections 11a, 12a shown to be opposed to a figure viewer are defined by a first surface sheet 21 (See Fig. 3 also) to be in contact with the user's face. The non-moisturizing section 15 is thinner than both the right inner moisturizing section 11a and the right outer moisturizing section 12a so that these moisturizing sections 11a, 12a protrude from the level defined by the non-moisturizing section 15 toward user's face, i.e., protrude upward as viewed in Fig. 2. Referring now to Fig. 2, a second surface sheet 22 (See Fig. 3 also) lying on the opposite side of the first surface sheet 21 to define a lower surface as viewed in Fig. 2 is to be in contact with the user's clothes and substantially flat in the moisturizing sections 11a, 12a as well as in the non-moisturizing section 15.

Referring to Fig. 3, the right outer side moisturizing section 12a comprises, between the first and second surface sheets 21, 22, a temperature regulating sheet 24 formed with a plurality of through-holes 23, a heat generating element 26 and a heat insulating sheet 27 laminated in this order as shown. The first surface sheet 21 comprises a breathable sheet 21a allowing oxygen permeation from the outer side toward the inner side and moisture permeation from the inner side toward the outer side through the right outer side moisturizing section 12a and short fibers 21b electrostatically implanted on this breathable sheet 21a. The breathable sheet 21a may be selected from a group including point-bonded nonwoven fabrics and spun-bonded nonwoven fabrics and the short fibers 21b may be those having a length in a range of 0.5 to 2 mm. The temperature regulating sheet 24 may be selected from a group including a plastic sheet foamed with closed cells and a laminate composed of a plurality of paper sheets or plastic film layers, in any case, formed with a plurality of through-holes each having a diameter in a range of 0.5 to 3 mm. The heat generating element 26 at least contains therein metal powders such as iron powders or aluminum powders adapted to be oxidized in the presence of oxygen supplied through the first surface sheet 21 and the temperature regulating sheet 24 and to generate a heat of oxidation, water essential to such an oxidation, and a water retaining material adapted to retain and to diffuse the water within the heat generating element 26, for example, pulp, wood flour or vermiculite. The heat generating element 26 may further comprise active charcoal serving as an accelerator, fibers and a binder serving to shape the heat generating element 26 in a sheet-like configuration. It is also possible to cover the heat generating element 26 with an air-permeable sheet material such as an air-permeable nonwoven fabric. The heat insulating sheet 27 may be selected from a group including a sheet foamed with closed-cells and a plastic film. The heat insulating sheet 27 functions to prevent heat and/or moisture vapor from escaping outward through the second surface sheet 22. The second surface sheet 22 serves to improve a touch of the pad 1 and may be selected from a group including a nonwoven fabric, a plastic film and a composite sheet comprising a nonwoven fabric defining an outer side and a plastic film defining an inner side of this composite sheet.

While the right outer side moisturizing section 12a of the pad 1 has been described above, the other moisturizing sections, i.e., each of the right inner side moisturizing section 11a, the left inner side moisturizing section 11a, the left inner side moisturizing section 11b and the left outer side moisturizing section 12b has the same cross-sectional construction as that of the right outer side moisturizing section 12a. The non-moisturizing section 15 shown in Figs. 1 and 2 is devoid of the heat generating element 26 and comprises the respective sheet materials shown in Fig. 3, i.e. , the first surface sheet 21, the temperature regulating sheet 24, the heat insulating sheet 27 and the second surface sheet 22 compressed and welded together under heating to a desired thickness as seen in Fig. 2. The non-moisturizing section 15 is more easily folded than the respective moisturizing sections 11a, 11b, 12a, 12b.

The moisturizing pad 1 constructed in the manner as has been described is protected by an air-impermeable wrap from being exposed to oxygen ambient air. Upon being unwrapped by a user, the first surface sheet 21 is put on the user's face 5 in a manner that the ventilator 16 may be aligned with a bridge of the user's nose. The moisturizing pad 1 is folded along the non-moisturizing section 15, the respective moisturizing sections 11a, 11b 12a, 12b gently bow so as to follow the contour of the user's face, and the right and left outer side of the moisturizing sections 12a, 12b partially come in contact with the lateral regions of the user's face 5. In the course of putting the moisturizing pad 1 on the user's face 5, oxygen in the ambient air permeates the first surface sheet 21 to the heat generating element 26. Thereupon, the metal powders within the heat generating element 26 such as iron powders is oxidized by the oxygen in the presence of salt and water and generates oxidation heat. The water within the heat generating element 26 is vaporized by the oxidation heat to generate moisture vapor which is, in turn, supplied through the temperature regulating sheet 24 and the first surface sheet 21 to the user's face 5. In this way, the user's face 5 is appropriately warmed and moisturized. While the moisture vapor generated from the heat generating element 26 may sometimes reach a temperature as high as in order of 80°C, such a high temperature of the moisture vapor can be controllably lowered to a desired surface temperature in order of 50°C in the course of permeating the porous temperature regulating sheet 24. Immediately after permeating the first surface sheet 21, the moisture vapor is diffused among the plurality of short fibers 21b and supplied to the user's face 5 over a wide area. Since the heat insulating sheet 27 and the second surface sheet 22 are air-impermeable, the moisture vapor cannot escape outward through the second surface sheet 22.

So far as the pad 1 adapted to be put on the user's face 5, it has been found by the inventors that the surface temperature of the first surface sheet 21 of the moisturizing pad 1 during generation of the moisture vapor is preferably in a range of 40 to 53°C and more preferably in a range of 48 to 53°C. For measurement of the surface temperature of the pad 1, a temperature measuring device JTG-6300 equipped with THERMOGRAPH-CAMERA manufactured by JEOL Ltd. was used and the measurement was carried out under a non-contact manner with the first surface sheet 21 and an environment condition of 20±1°C and 50 % R.H. Preferably the surface temperature of the moisturizing pad 1 to a desired level as soon as possible after the pad 1 has been unwrapped and, specifically, the surface temperature rises preferably to a level of 40 to 53°C, more preferably to a level of 48 to 53°C within 3 minutes after the pad 1 has been unwrapped. However, it is undesirable that the surface temperature rises to 54°C or higher or does not rise to 40°C. In this manner, the properties of the moisturizing pad 1 should be evaluated also with respect to the time required until the surface temperature rises to 40°C after the pad 1 has been unwrapped. Specifically, the time required is preferably 3 minutes or less and more preferably 2 minutes or less. The time required exceeding 3 minutes is undesirable. The properties of the moisturizing pad 1 should be evaluated also with respect to the time duration for which the surface can be maintained at a desired temperature. Specifically, a time duration for which the pad is maintained at a level in order of 40°C is preferably 5 minutes or longer. Furthermore, the properties of the moisturizing pad 1 should be evaluated also with respect to a quantity of moisture vapor generated from the pad 1. Specifically, the quantity of moisture vapor generated for 20 minutes after the pad 1 has been unwrapped is preferably 4 g or more and more preferably 5 g or more. The quantity of moisture vapor can be determined by a difference of the weights of the moisturizing pad 1 measured immediately after the pad 1 has been unwrapped and measured after 20 minutes have elapsed. According to the present invention the preferred heat generating element 26 of each of the moisturizing sections 11a, 11b, 12a, 12b is impregnated 18 - 22 % by weight of saline including 17.5 - 26 % by weight of salt.

### EXAMPLES

Properties of the pad 1 shaped as shown in Fig. 1 were evaluated using the right outer side moisturizing section 12a. This moisturizing section 12a has a dimension C of 80 mm, a dimension D of 112 mm, and an area of the first surface sheet 21 of 45.4 cm². The moisturizing section 12a had the cross-sectional construction as shown in Fig. 3 and the first surface sheet 21 comprising a nonwoven fabric having a basis weight in a range of 90 to 97 g/m² and a thickness in a range of 1.5 to 2.5 mm. As the temperature regulating sheet 24, a foamed polyethylene sheet was used, which had a basis weight of 73 g/m², a thickness of 3 mm, a foaming ratio of 30 and an open area ratio of the through-holes 23 to the lower surface of the sheet 24 being 30 %. As the heat generating element 26, the base material comprising in addition to 95 % by weight of iron powders, 5 % by weigh of additives such as vermiculite and wood flour serving as the water retaining material and active charcoal serving for acceleration of oxidation was impregnated with various amounts of saline solution having a concentration of 20 % by weight of salt to obtain respective samples of the moisturizing section 12a. The individual samples were air-tightly wrapped with polyethylene bags laminated with aluminum foil in order to avoid a contact with oxygen. Respective properties of the moisturizing section after unwrapped were evaluated and the result of this evaluation is shown in TABLE 1.

**TABLE 1**

| | **EXAMPLE 1** | **EXAMPLE 2** | **EXAMPLE 3** | **EXAMPLE 4** | **EXAMPLE 5** | **CONTROL 1** | **CONTROL 2** | **CONTROL 3** |
|---|---|---|---|---|---|---|---|---|
| **weight of moisturizing element (g)** | 15.0 | 16.0 | 16.9 | 12.6 | 12.7 | 13.1 | 14.2 | 15.4 |
| **impregnating percentage with saline solution (%)** | 20.0 % | 17.5 % | 19.5 % | 23.0 % | 26.0 % | 26.7 % | 31.0 % | 14.9 % |
| **rising rate of temperature (min)** | ○ | ○ | ○ | ○ | ○ | × | × | ○ |
| **Duration (min)** | ○ | ○ | ○ | ○ | ○ | ○~× | ○~× | ○ |
| **Maximum temperature (°C)** | ○ | ○ | ○ | ○ | ○ | ○~× | ○~× | × |
| **Temperature after 3 min (°C)** | ○ | ○ | ○ | ○ | ○ | ○~× | ○~× | ○ |
| **quantity of generated moisture vapor (g)** | **6** | **5.5** | **5.5** | **4.6** | **4.4** | **2.8** | **2.4** | **5.6** |

In TABLE 1, "rising rate of temperature" refers to a time (min) required until a temperature of the heat generating element rises to 40°C and a mark ○ indicates that this time was 2 minutes or less, and a mark × indicates that this time was 3 minutes or more. "duration" refers to a time (min) required until a temperature of the heat generating element lowered from 40°C after the temperature of this element had once reached 40°C. a mark ○ indicates that this time was 5 minutes or more, a mark × indicates that this time was less than 5 minutes. "maximum temperature" refers to a maximum temperature (°C) observed during the evaluation for about one hour. A mark ○ indicates that this maximum temperature was in a range of 48 to 53°C, and a mark × indicates that this maximum temperature was lower than 40°C or 54°C or higher. In "temperature 3 minutes after unwrapped", a mark ○ indicates that this temperature was in a range of 48 to 53°C, and a mark × indicates that this temperature was lower than 40°C or 54°C or higher. "quantity of generated moisture vapor" refers to a quantity of moisture vapor (g) generated for 20 minutes. This amount is preferably 3 g or more and more preferably 5 g or more.

TABLE 1 indicates also the result of evaluation conducted on CONTROLS 1 through 3. Moisturizing sections impregnated with saline solution at a ratio of 26 % by weight or higher or at a ratio of 15 % or lower were used as these CONTROLS.

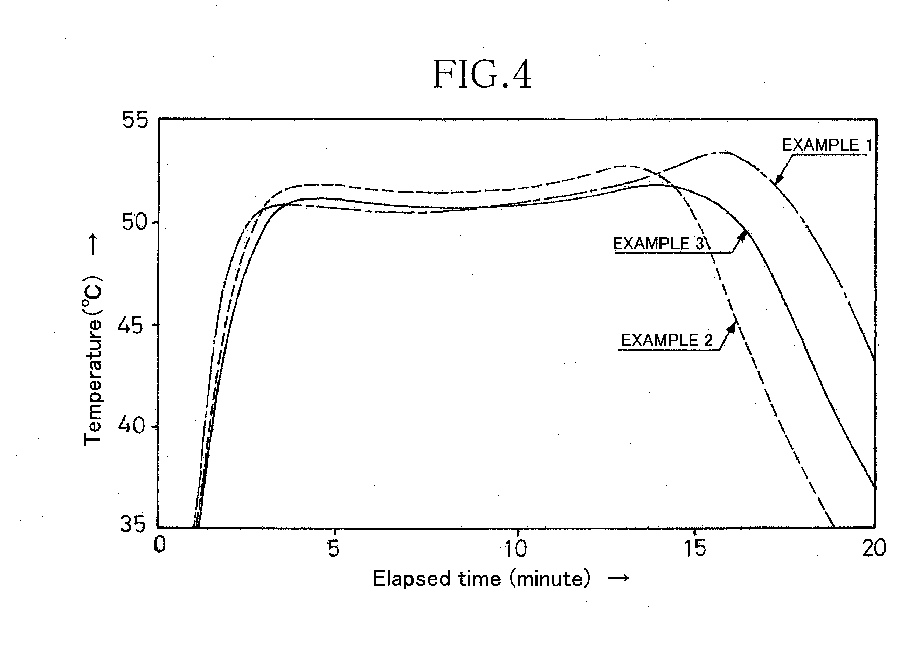

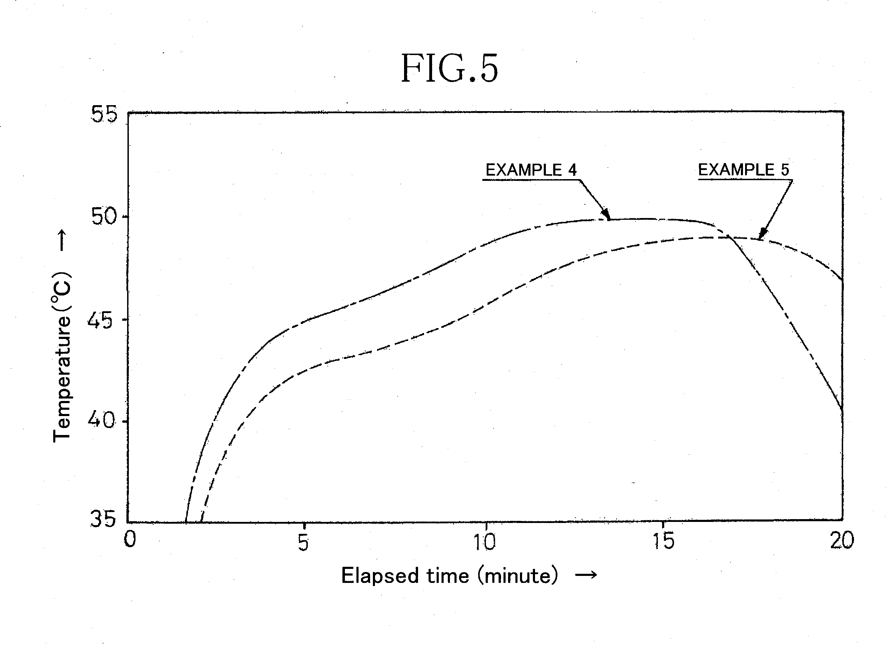

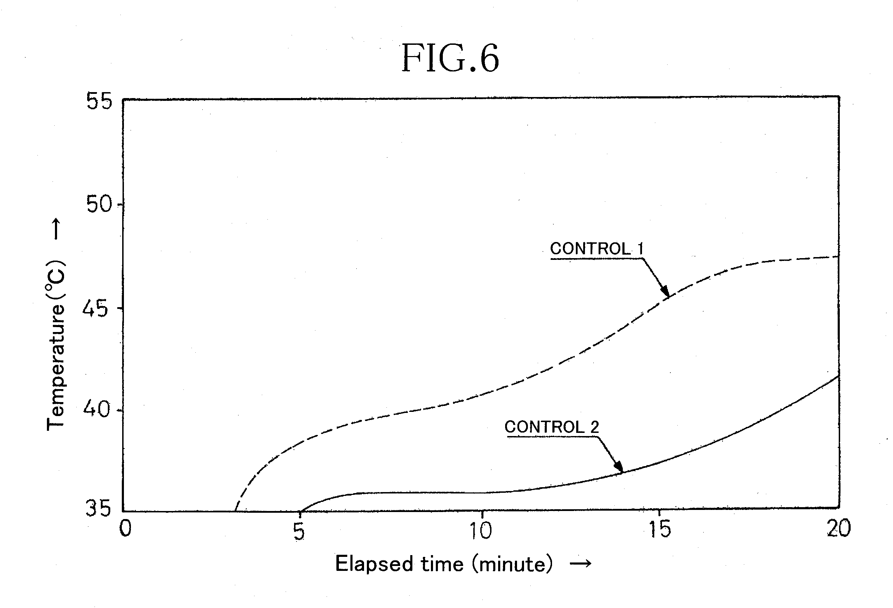

Figs. 4, 5 and 6 are graphic diagrams plotting a time elapsed after unwrapping versus a surface temperature of the moisturizing section measured on EXAMPLES 1 - 3, 4 and 5, CONTROLS 1 - 3, respectively.

## Claims

1. A pad comprising:
a first surface sheet to be put in contact with a user's skin;
a second surface sheet lying on a side opposite to said first surface sheet;
a heat generating element sandwiched between said first and second surface sheets and containing therein at least metal powders, salt, water and water retaining materials;
of said first and second surface sheets, at least said first surface sheet being air-permeable and said pad is wrapped with an oxygen-impermeable sheet so that said water may be vaporized under an oxidation heat generated in said heat generating element to moisturize said user's skin as said pad is unwrapped whereupon said heat generating element is supplied with oxygen through said first surface sheet; said pad being **characterized in that**:
said salt and said water are provided in a form of saline solution with which said heat generating element is impregnated at a ratio in a range of 17.5 to 26 % by weight with respect to said heat generating element and said saline solution has a saline concentration in a range of 18 to 22 % by weight.

2. The pad according to Claim 1, wherein at least 4 g of said moisture vapor is generated from said pad until 20 minutes elapse after said pad has been unwrapped in a room at a temperature of 20±1°C.

3. The pad according to Claim 1 or 2, wherein a temperature of said pad on the side of said first surface sheet rises to 40°C within 3 minutes after said pad has been unwrapped in a room at a temperature of 20±1°C.
